# FASCICULE DE BREVET EUROPEEN

(11) **EP 1 117 410 B1**
(45) Date de publication et mention de la délivrance du brevet: **28.12.2005**
(21) Numéro de dépôt: 99946282.3
(22) Date de dépôt: 01.10.1999
(51) Int. Cl.: A61K 31/66, A61K 31/675, A61P 43/00

(54) **UTILISATION DE DERIVES DE L'ACIDE BISPHOSPHONIQUE POUR LA PREPARATION D'UN MEDICAMENT DESTINE AU TRAITEMENT DES BOITERIES**
VERWENDUNG VON BISPHOSPHONSAÜRE DERIVATEN ZUR HERSTELLUNG EINES ARZNEIMITTELS ZUR BEHANDLUNG VON LAHMEN
USE OF BISPHOSPHONIC ACID DERIVATIVES FOR PREPARING A MEDICINE FOR TREATING LAMENESS

(30) Priorité: 02.10.1998 FR 9812388
(43) Date de publication de la demande: 25.07.2001
(73) Titulaire: CEVA SANTE ANIMALE, 33501 Libourne Cedex (FR)
(72) Inventeur: DU MESNIL, Philippe, F-33000 Bordeaux (FR); BARDON, Thierry, F-33270 Bouliac (FR); THIBAUD, Dominique, F-338000 Bordeaux (FR)
(74) Mandataire: Bernasconi, Jean Raymond
(86) Numéro de dépôt international: PCT/FR1999/002349
(87) Numéro de publication internationale: WO 2000/020008

(56) Documents cités:
- WO-A-97/12619
- LEPAGE ET AL: "l'emploi d'un bisphosphonate (ADP) dans la prévention des exostoses chez le poney Shetland. Etude préliminaire" ANN. MED. VET., vol. 132, 1988, pages 391-399, XP000614171

## Description

La présente invention a pour objet l'utilisation de dérivés de l'acide bisphosphonique pour la préparation de médicaments destinés au traitement des boiteries apparaissant lors d'ostéo-arthrose, d'ostéochondrose, ou d'enthésopathies des insertions osseuses des tendons, des ligaments ou des aponévroses en médecine vétérinaire.

Par boiterie, on entend une démarche irrégulière provoquée par la perception d'une douleur par appui partiel ou total sur un ou plusieurs membres pendant le fonctionnement des membres sollicités.

Une boiterie peut se manifester cliniquement de façon intermittente ou continue pendant plusieurs jours, plusieurs semaines ou plusieurs mois.

Les boiteries résultent plus précisément de l'apparition de lésions douloureuses au niveau de la structure osseuse, des cartilages, des ligaments, de la membrane synoviale, du tissu conjonctif ou d'une anomalie de la vascularisation locale. Ainsi, les boiteries sont généralement associées à une ou plusieurs des composantes suivantes :
- une composante osseuse qui est le résultat d'une modification de l'architecture osseuse et/ou des cartilages de croissance des os sièges de la boiterie, telle que, par exemple, des pertes de substance osseuse, la formation de kystes, une déformation de l'os ou des épaississements excessifs des cartilages de croissance;
- une composante articulaire qui est le résultat d'une modification de la structure des cartilages articulaires telles que, par exemple, des érosions des surfaces cartilagineuses et/ou par modification de la membrane synoviale et/ou d'une modification des ligaments articulaires;
- une composante musculaire qui est le résultat d'une modification du développement musculaire telle que, par exemple, une atrophie musculaire; et
- une composante vasculaire qui est le résultat d'une modification de la vascularisation locale, telle que, par exemple, une réduction de la vascularisation de la région lésée.

L'invention vise à fournir des médicaments utilisables dans le traitement de boiteries apparaissant lors d'ostéo-arthrose, d'ostéochondrose, ou d'enthésopathies des insertions osseuses des tendons, des ligaments ou des aponévroses chez l'animal ne souffrant ni de fractures, ni d'arthrite, ni d'exostoses.

Parmi les facteurs susceptibles de déclencher de telles boiteries, on peut citer les sollicitations mécaniques constantes et/ou intenses de l'appareil locomoteur. Chez un animal peu préparé à l'exercice physique, l'intensité de la sollicitation mécanique capable de déclencher une boiterie pourra être relativement faible.

A l'inverse, chez un animal préparé à l'exercice physique, la boiterie apparaîtra lorsqu'il sera soumis à une sollicitation mécanique dont la force ou le caractère répétitif dépassera les capacités de résistance de ses membres. Certaines espèces animales sont plus particulièrement enclines à développer ce type de boiteries. C'est notamment le cas des équidés.

De fait, chez le cheval, l'appareil locomoteur est plus fréquemment sollicité que celui d'autres espèces animales que ce soit lors de compétitions sportives ou lorsqu'il est utilisé comme monture par l'homme. Dans cette espèce animale, les boiteries représentent une des principales entités cliniques nécessitant une consultation vétérinaire. Leur description clinique est bien connue; une revue assez exhaustive en est présentée dans le livre "Les boiteries du cheval" édité sous la direction d'O.R. ADAMS (Editions Maloine, 1990). Parmi les boiteries les plus fréquentes, on peut citer l'éparvin osseux ou ostéoarthrose de l'étage distal du tarse du cheval, les ostéochondroses du cheval, les enthésopathies des insertions osseuses des tendons, des ligaments ou des aponévroses du cheval.

Ainsi, selon un mode de réalisation préféré, l'invention concerne l'utilisation de dérivés de l'acide bisphosphonique pour la préparation de médicaments destinés au traitement de boiteries apparaissant lors d'ostéo-arthrose, d'ostéochondrose, ou d'enthésopathies des insertions osseuses des tendons, des ligaments ou des aponévroses, chez le cheval ne souffrant ni de fractures, ni d'arthrite, ni d'extostoses.

Plus généralement, l'invention concerne l'utilisation de dérivés de l'acide biphosphonique pour la préparation de médicaments destinés au traitement des boiteries apparaissant lors d'ostéo-arthrose, d'ostéochondrose, ou d'enthésopathies des insertions osseuses des tendons, des ligaments ou des aponévroses chez l'animal ne souffrant ni d'arthrite, ni de fractures, ni d'extostoses.

Par extostose, on entend une forme particulière d'ossification ectopique.

Les dérivés de l'acide bisphosphonique utilisables dans le cadre de l'invention ont pour formule générale : 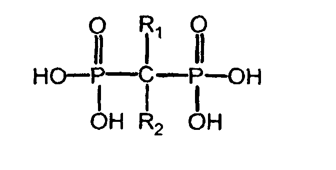 dans laquelle :
- R₁ représente un atome d'hydrogène, un atome d'halogène, un hydroxyle, un amino, un monoalkyl(C₁-C₄)amino, un dialkyl(C₁-C₄)amino;
- R₂ représente un atome d'halogène, un alkyle linéaire comportant de 1 à 5 atomes de carbone non substitué ou substitué par un groupe choisi parmi un atome de chlore, un hydroxyle, un amino, un monoalkyl(C₁-C₄)amino, un dialkyl(C₁-C₄)amino; un cycloalkyl(C₃-C₇)amino,
ou R₂ représente un phénoxy, un phényle, un thiol, un phénylthio, un chlorophénylthio, un pyridyle, un pyridylméthyle, un 1-pyridyl-1-hydroxyméthyle, un imidazolylméthyle, un thiomorpholin-4-yle.

Les sels de ces composés avec des acides ou des bases minéraux ou organiques pharmaceutiquement acceptables sont également utilisables dans le cadre de l'invention. Des exemples de sels avec des acides sont les chlorhydrate, bromhydrate, sulfate, acétate, hydrogénosulfate, dihydrogénophosphate, méthanesulfonate, méthylsulfate, maléate, fumarate, sulfonate, 2-naphtalènesufonate, glycolate, gluconate, citrate, iséthionate, benzoate, salicylate, ascorbate, tartrate, succinate, lactate, glutarate, toluènesulfonate et ascorbate. Comme exemple de sels avec des bases minérales ou organiques, on peut citer les sels d'ammonium ou les sels de métaux alcalins, tels que par exemple les sels de sodium.

Les hydrates de ces composés sont de même utilisables selon l'invention.

Ces composés sont notamment décrits dans EP 623 347.

Parmi ces dérivés d'acide bisphosphonique, on peut citer en particulier les composés suivants :
- l'acide 1-hydroxy-éthylidène bisphosphonique dont la dénomination commune internationale est l'acide étidronique, et ses sels de sodium;
- l'acide 2-pyridin-2-yl-éthylidène bisphosphonique dont la dénomination commune internationale est l'acide piridronique, et ses sels de sodium;
- l'acide dichlorométhylène bisphosphonique dont la dénomination commune internationale est l'acide clodronique, et ses sels de sodium;
- l'acide 3-amino-1-hydroxy-propylidène bisphosphonique dont la dénomination commune internationale est l'acide pamidronique, et ses sels de sodium;
- l'acide 4-amino-1-hydroxy-butylidène bisphosphonique dont la dénomination commune internationale est l'acide alendronique, et ses sels de sodium;
- l'acide 6-amino-1-hydroxy-hexylidène bisphosphonique et ses sels;
- l'acide phénoxyméthylène bisphosphonique et ses sels;
- l'acide thiomorpholinométhylène bisphosphonique et ses sels;
- l'acide 4-chloro-phénylthiométhylène bisphosphonique dont la dénomination commune internationale est acide tiludronique et ses sels pharmaceutiquement acceptables, notamment le sel disodique;
- l'acide 1-hydroxy-2-(pyridin-3-yl)éthylidène bisphosphonique dont la dénomination commune internationale est acide risédronique et ses sels de sodium;
- l'acide 1-hydroxy-2-(imidazol-2-yl)éthyl-1,1- bisphosphonique et ses sels;
- l'acide (cycloheptylamino)méthylène bisphosphonique et ses sels;
- l'acide 2-hydroxyéthylidène-2-(pyridin-3-yl)-1,1- bisphosphonique et ses sels de sodium.

Selon la présente invention, l'usage de l'acide tiludronique et de ses sels pharmaceutiquement acceptables, en particulier le sel disodique, ou de ses hydrates est particulièrement préféré.

Les dérivés de l'acide bisphosphonique sont connus pour inhiber la résorption osseuse et diminuer l'activité des ostéoclastes, ainsi qu'il résulte notamment des articles suivants :
- « Diphosphonates inhibit hydroxyapatite dissolution in vitro and bone resorption in tissue culture and in vivo », FLEISCH H., RUSSELL R., FRANCIS M., Science, 1969, 165, 1262-1264
- « Two modes of action of bisphosphonates on osteoclastic résorption of mineralized matrix », BOONEKAMP P.M., VAN DER WEE-PALS L.J.A., Van WIJK-LENNEP, THESING C.W., BIJVOET O.L.M., Bone Miner., 1986,1, 27-39
- « Dichloromethylene bisphosphonate (Cl₂MBP) inhibits bone résorption through injury to osteoclasts that resorb CIMBP coated bone », FLANAGHAN A.M., CHAMBERS T.J., Bone Miner., 1989, 6, 33.

De nombreux dérivés de l'acide bisphosphonique sont en développement ou déjà commercialisés en médecine humaine dans le traitement d'affections osseuses. Une revue des utilisations thérapeutiques de ces dérivés est présentée dans le livre « Bisphosphonate on bones » édité sous la direction de BIJVOET O.L.M., FLEISCH H.A., CANFIELD R.E. et RUSSELL R.G.G. (Elsevier Science BV, 1995). Les utilisations principales concernent le traitement d'affections osseuses telles que la maladie de Paget ou l'ostéoporose. Les autres utilisations usuellement décrites visent le traitement de l'hypercalcémie maligne, des tumeurs osseuses ou des métastases osseuses. On a également pu mettre en évidence l'activité anti-inflammatoire de certains dérivés de l'acide bisphosphonique dans un modèle d'arthrite, chez le rat, provoquée par injection de mycobacterium.

Cependant les inventeurs n'ont pas connaissance de travaux démontrant l'activité anti-inflammatoire de ces dérivés de l'acide bisphosphonique dans des pathologies distinctes de l'arthrite ou plus généralement chez d'autres espèces. Plus récemment, il a été décrit l'intérêt de certains dérivés de l'acide bisphosphonique dans l'amélioration de la réparation des fractures. On se rapportera notamment à EP 600 834 ou US 5488041.

D'autre part, WO 97 126 19 décrit l'utilisation de dérivés d'acide bisphosphonique pour traiter la maladie naviculaire chez le cheval.

Enfin, il a été démontré dans Ann. Med. Vet. vol. 132, 198, p. 391-399, que le 3-amino-1-hydroxypropylidène-1,1-bis-phophonate est efficace dans la prévention des exostoses. Dans cette étude, l'ossification induite par soulèvement du périoste a été choisie comme modèle expérimental. Bien que la présence d'exostoses puisse provoquer une boiterie chez l'animal, cette activité du 3-amino-1-hydroxypropylidène-1,1-bis-phosphonate ne suggère nullement l'efficacité des phosphonates à traiter les boiteries apparaissant lors d'ostéoarthroses, d'ostéochondroses ou d'enthésopathies.

Dans un autre domaine, il est également décrit l'usage des dérivés de l'acide bisphosphonique dans le diagnostic par scintigraphie de certaines affections osseuses. Un exemple d'une telle utilisation est rapporté par KEEGAN K.G., WILSON D.A., LATTIMER C.L., TWARDOCK A.R., ELLERSIECK M.R. (Am. J. Vet. Res., 1996, 57, 415-421) dans l'évaluation scintigraphique du marquage par le ^{99m}Tc-méthylène diphosphonate de la région naviculaire chez les chevaux présentant une boiterie localisée au pied sans qu'il soit fait mention du bénéfice clinique apporté par la fixation du dérivé de l'acide bisphosphonique dans les os de la région palmaire.

Les traitements médicaux les plus usuellement prescrits lors de boiteries visent à soulager la douleur ; c'est le cas, par exemple, des traitements par des médicaments anti-inflammatoires non stéroïdiens. Cependant, ces traitements sont insatisfaisants dans la mesure où ils ne soignent pas les lésions à l'origine de l'inflammation. Leur efficacité est par ailleurs limitée à la période d'administration, les effets bénéfiques des anti-inflammatoires disparaissant dès la fin du traitement. Le repos est également souvent préconisé pour permettre le retour à un état normal de la région de l'appareil locomoteur à l'origine de la boiterie. De façon plus spécifique, chez le cheval, l'usage de ferrures orthopédiques est conseillé. Plus particulièrement encore dans cette espèce animale, l'usage de composés modifiant la vascularisation de la région naviculaire est préconisé dans le traitement de la maladie naviculaire ; c'est ainsi qu'est prescrit l'isoxsuprine, agent vasodilatateur par GABRIEL A., CAUDRON I., SERTEYN D., COLLIN B. dans « Syndrome naviculaire : anatomie, étiopathogénie, diagnostic, traitement », Ann. Med. Vet., 1994, 138, 309-330).

De façon surprenante, il a été trouvé que les dérivés de l'acide bisphosphonique sont utiles dans le traitement des boiteries apparaissant lors d'ostéo-arthrose, d'ostéochondrose, ou d'enthésopathies des insertions osseuses des tendons, des ligaments ou des aponévroses. Il a été montré de façon tout-à-fait inattendue que l'utilisation de ces dérivés permettait une amélioration significative et durable des signes cliniques de boiterie, voire une guérison de la boiterie, même au-delà de la période de traitement, et ceci sans qu'une augmentation de la densité osseuse n'ait pu être identifiée par examen radiologique.

Ainsi, les effets bénéfiques observés ne seraient pas liés à l'activité inhibitrice de la résorption osseuse des dérivés de l'acide bisphophonique.

De tels médicaments sont utilisables en médecine vétérinaire.

Ils peuvent être administrés par différentes voies d'administration, par exemple par voie parentérale, par voie orale, par voie rectale, par voie intra-articulaire, par voie cutanée ou par voie transcutanée ou transdermique.

Le mode d'administration de tels médicaments est déterminé en fonction de l'espèce traitée, de l'âge, du poids et de la sévérité de la pathologie.

Le rythme d'administration peut consister en une administration unique ou en administrations répétées. Dans le cas d'administrations répétées, le traitement peut être administré de façon continue ou de façon intermittente. Lorsque l'on choisira un traitement continu, le rythme d'administration préféré pourra être d'une administration quotidienne unique à 3 administrations quotidiennes pendant une période pouvant varier de quelques jours à quelques mois. Lorsque l'on choisira un traitement par intermittence, on pourra adopter l'un des rythmes d'administration suivants : une administration tous les 2 ou 3 jours, une administration hebdomadaire, bi-mensuelle ou mensuelle pendant des périodes pouvant varier de quelques semaines à quelques mois.

La concentration du médicament en dérivé de l'acide bisphosphonique dépend de l'activité et de la durée d'action de ce dérivé, du mode d'administration, de l'âge, du poids, du sexe, de l'importance de l'effet recherché, de l'espèce de destination ou, pour certaines espèces animales, de la race.

Pour des préparations liquides à usage parentéral ou oral, la concentration du médicament en dérivé d'acide bisphosphonique peut être comprise entre 0,001 % et 90 % en rapport poids/volume. Pour des préparations destinées à la voie orale, elle peut être comprise entre 0,001 mg et 10 g par unité de dosage.

Le médicament peut également se présenter sous la forme d'implant.

Les doses lors de chaque administration des médicaments préparés selon la présente invention, exprimées par rapport au poids corporel, peuvent varier entre 0,001 mg/kg et 100 mg/kg.

A titre d'exemple, on pourra administrer au cheval, par voie intraveineuse, des doses de 0,01 mg/kg/semaine à 1 mg/kg/semaine d'acide tiludronique ou d'un de ses sels.

Pour une administration par voie orale, la composition pharmaceutique selon l'invention peut être sous forme de comprimé, gélule, poudre, granulé, gouttes ou toute autre forme administrable par voie orale.

La composition selon l'invention peut contenir en outre des ingrédients utilisés habituellement en pharmacie pour la préparation de formes orales. Ainsi la composition selon l'invention peut contenir un agent de désintégration, un agent d'écoulement, un lubrifiant et tout excipient de masse convenable.

Comme excipient de masse, on peut utiliser le lactose, la cellulose ou les amidons. Comme lubrifiant, on peut utiliser l'acide stéarique, le stéarate de magnésium, la L-leucine ou, par exemple, le tribéhénate de glycérol. Comme agent de désintégration, on peut utiliser le carboxyméthylamidon sodique, la carboxyméthylcellulose sodique réticulée ou, par exemple, la polyvinylpyrrolidone réticulée. Comme agent d'écoulement, on peut utiliser la silice pure ou le dioxyde de silicium colloïdal.

La présente invention se réfère également aux formes orales à dissolution instantanée et aux formes orales effervescentes obtenues en ajoutant un couple effervescent à la composition selon l'invention. Comme couple effervescent, on peut utiliser, l'acide tartrique et le bicarbonate de sodium ou l'acide citrique et le bicarbonate de sodium.

L'invention se réfère également aux comprimés à dissolution instantanée, aux comprimés effervescents ainsi qu'aux comprimés recouverts d'un enrobage. Une composition contenant du laurylsulfate de sodium selon le brevet européen EP 336851 est particulièrement convenable.

Pour une administration rectale, on recourt à des suppositoires qui sont préparés avec des liants fondant à la température rectale, par exemple du beurre de cacao ou des polyéthylèneglycols.

Les préparations injectables sont préparées par mélange d'un ou plusieurs dérivés de l'acide bisphosphonique avec un régulateur de pH, un agent tampon, un agent de mise en suspension, un agent de solubilisation, un stabilisant, un agent de tonicité et/ou un conservateur, et par transformation du mélange en une injection intraveineuse, sous-cutanée ou intramusculaire, selon un procédé classique. Le cas échéant, les préparations injectables peuvent être lyophilisées selon un procédé dassique.

Des exemples d'agents de mise en suspension englobent la méthylcellulose, le polysorbate 80, l'hydroxyéthylcellulose, l'acacia, la gomme adragante en poudre, la carboxyméthycellulose sodique et le monolaurate de sorbitane polyéthoxylé.

Des exemples d'agent de solubilisation englobent l'huile de ricin solidifiée par du polyoxyéthylène, le polysorbate 80, le nicotinamide, le monolaurate de sorbitane polyéthoxylé, le macrogol et l'ester éthytique d'acide gras d'huile de ricin.

En outre, le stabilisant englobe le sulfite de sodium, le métalsulfite de sodium et l'éther, tandis que le conservateur englobe le p-hydroxybenzoate de méthyle, le p-hydroxybenzoate d'éthyle, l'acide sorbique, le phénol, le crésol et le chlorocrésol.

Un exemple d'agent de tonicité est le mannitol.

Lors de la préparation des solutions ou suspensions injectables, il est souhaitable de veiller à ce qu'elles soient isotoniques au sang.

### Exemple 1

Une étude clinique a été réalisée pour évaluer les effets d'un traitement par le tiludronate disodique de chevaux présentant une boiterie lors d'éparvin osseux (qui est l'ostéo-arthrose de l'étage distal du tarse).

L'étude a été conduite sur 5 chevaux, le diagnostic de l'ostéo-arthrose à l'origine de la boiterie reposant sur les critères suivants :
- des signes locaux dans la région du jarret : chaleur, tuméfaction, douleur
- des signes fonctionnels de boiterie notés sur une échelle de 0 (aucun signe de boiterie) à 4 (défaut d'appui au repos et/ou en mouvement et difficultés de déplacement)
- un test fonctionnel de locomotion après flexion du jarret jugé également sur une échelle de 0 (locomotion non modifiée par la flexion) à 4 (départ en suppression d'appui)
- un examen radiologique pour juger qualitativement des signes radiologiques dans la région du jarret.

Pour être inclus, l'animal ne devait pas présenter de signes ou de lésions d'arthrite septique, ni de fractures des os de la région du jarret, ni même présenter d'exostose dans la région du jarret. Il ne devait pas non plus avoir reçu une injection de traitement anti-inflammatoire dans les articulations de la région du jarret.

Le port d'une ferrure orthopédique n'était pas contre-indiqué dans la mesure où la plupart des animaux présentaient une boiterie depuis plusieurs semaines et étaient déjà porteurs d'une ferrure de ce type.

Tous les animaux ont reçu comme unique traitement le tiludronate disodique sous forme d'une solution injectable administrée par voie intraveineuse, à la dose de 0,1 mg/kg, à raison d'une administration hebdomadaire pendant 6 à 10 semaines. Le premier jour de traitement correspond à J0.

Pour apprécier les effets du traitement, les animaux ont été revus 1 mois après J0 (M1), 2 mois après J0 (M2) et 6 mois après J0 (M6). Les effets du traitement ont été jugés sur :
- les signes locaux, les signes fonctionnels et le test de locomotion après flexion comme définis lors de l'inclusion de l'animal dans l'essai
- un examen radiologique pour évaluer l'évolution des signes radiologiques
- un jugement sur la reprise de l'activité sportive de l'animal.

La prise en compte de l'ensemble de ces critères permettaient de placer le cheval dans l'une des catégories suivantes à l'issue des 3 examens de contrôle à M1, M2 et M6:
- catégorie « Très favorable » : absence de signes locaux, note 0 sur les signes et le test fonctionnels ; l'animal a repris une activité comparable à celle qu'il avait avant de boiter
- catégorie « Favorable » : les signes locaux sont très atténués, les signes fonctionnels ont été améliorés de 1 ou 2 degrés, le cheval est prêt à reprendre une activité physique comparable à celle qu'il avait avant de boiter
- catégorie « Moyenne » : persistance de signes locaux, signes fonctionnels améliorés de 1 degré ; persistance d'une gène locomotrice ne permettant pas de retrouver une activité physique normale
- catégorie « Médiocre » : persistance des signes locaux, pas d'amélioration des signes fonctionnels
- catégorie « Nulle » : aucune amélioration des signes locaux ou fonctionnels voire aggravation des signes locaux et des signes fonctionnels.

Les résultats obtenus ont été les suivants (expression en pourcentage d'animaux présents dans chaque catégorie) :

| Catégorie | Examen à M1 | Examen à M2 | Examen à M6 |
|---|---|---|---|
| Très favorable | 0 % | 40 % | 40 % |
| Favorable | 40 % | 60 % | 20 % |
| Moyenne | 60 % | 0 % | 20 % |
| Médiocre | 0 % | 0 % | 0 % |
| Nulle | 0 % | 0 % | 20 % |

L'évolution de la boiterie provoquée par l'éparvin osseux est favorable à très favorable chez la majorité des animaux à l'issue du traitement. La persistance des effets bénéfiques est également très satisfaisante.

### Exemple 2

Une étude clinique a été réalisée pour évaluer les effets d'un traitement par le tiludronate disodique de chevaux présentant une boiterie associée à des lésions osseuses sous la forme de kystes sous-chondraux lors d'ostéochondrose.

Le protocole de l'essai ainsi que le suivi clinique des animaux ont été les mêmes que ceux décrits dans l'exemple 1 à la différence que les signes locaux, fonctionnels et radiologiques ont été évalués dans les articulations atteintes d'ostéochondrose.

5 chevaux ont été inclus dans l'essai.

Les résultats obtenus ont été les suivants (expression en pourcentages d'animaux présents dans chaque catégorie) :

| Catégorie | Examen à M1 | Examen à M2 | Examen à M6 |
|---|---|---|---|
| Très favorable | 40 % | 20 % | 60 % |
| Favorable | 0 % | 40 % | 0 % |
| Moyenne | 40 % | 20 % | 0 % |
| Médiocre | 0 % | 0 % | 40 % |
| Nulle | 20 % | 20 % | 0 % |

Ces résultats démontrent le bénéfice clinique apporté par l'administration du dérivé de l'acide bisphosphonique sur le traitement des boiteries associées à des lésions osseuses lors d'ostéochondrose.

### Exemple 3

Une étude clinique a été réalisée pour évaluer les effets d'un traitement par le tiludronate disodique de chevaux présentant une boiterie lors d'enthésopathie d'insertion des tendons et des ligaments.

Le protocole de l'essai ainsi que le suivi clinique des animaux ont été les mêmes que ceux décrits dans l'exemple 1 à la différence que les signes locaux, fonctionnels et radiologiques ont été appréciés dans la région des insertions tendineuses ou ligamentaires à l'origine de la boiterie.

6 chevaux ont été inclus dans cet essai.

Les résultats obtenus ont été les suivants (expression en pourcentage d'animaux présents dans chaque catégorie) :

| Catégorie | Examen à M1 | Examen à M2 | Examen à M6 |
|---|---|---|---|
| Très favorable | 0 % | 0 % | 40 % |
| Favorable | 50 % | 67 % | 40 % |
| Moyenne | 33 % | 33 % | 0 % |
| Médiocre | 17 % | 0% | 20 % |
| Nulle | 0 % | 0 % | 0 % |

Ces résultats démontrent le bénéfice clinique apporté par l'administration du dérivé de l'acide bisphosphonique sur le traitement des boiteries provoquées par les enthésopathies d'insertion.

## Revendications

1. Utilisation d'un dérivé de l'acide bisphosphonique de formule : 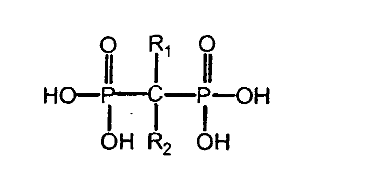 dans laquelle :
- R₁ représente un atome d'hydrogène, un atome d'halogène, un hydroxyle, un amino, un monoalkyl(C₁-C₄)amino, un dialkyl(C₁-C₄)amino;
- R₂ représente un atome d'halogène, un alkyle linéaire comportant de 1 à 5 atomes de carbone non substitué ou substitué par un groupe choisi parmi un atome de chlore, un hydroxyle, un amino, un monoalkyl(C₁-C₄)amino, un dialkyl(C₁-C₄)amino; un cycloalkyl(C₃-C₇)amino,
ou R₂ représente un phénoxy, un phényle, un thiol, un phénylthio, un chlorophénylthio, un pyridyle, un pyridylméthyle, un 1-pyridyl-1-hydroxyméthyle, un imidazolylméthyle, un thiomorpholin-4-yle
d'un de ses sels pharmaceutiquement acceptables ou d'un de ses hydrates dans la préparation d'un médicament destiné au traitement des boiteries apparaissant lors d'ostéo-arthrose, d'ostéochondrose, ou d'enthésopathies des insertions osseuses des tendons, des ligaments ou des aponévroses chez l'animal ne souffrant ni d'arthrite, ni de fractures, ni d'exostoses.

2. Utilisation selon la revendication 1, **caractérisée en ce que** l'animal appartient à la famille des équidés.

3. Utilisation selon la revendication 1, **caractérisée en ce que** l'animal est le cheval.

4. Utilisation selon l'une quelconque des revendications 1 à 3 pour la préparation d'un médicament contenant 0,001 mg à 10 g du dérivé de l'acide bisphosphonique par unité de dosage.

5. Utilisation selon l'une quelconque des revendications précédentes, **caractérisée en ce que** le médicament est une préparation liquide administrable par voir orale ou parentérale contenant de 0,001% à 90% en poids/volume du dérivé de l'acide bisphosphonique.

6. Utilisation selon l'une quelconque des revendications 1 à 4 pour la préparation d'un médicament administrable par voie orale.

7. Utilisation selon l'une quelconque des revendications 1 à 4 pour la préparation d'un médicament administrable par voie parentérale.

8. Utilisation selon l'une quelconque des revendications 1 à 4 pour la préparation d'un médicament administrable sous forme d'implant.

9. Utilisation selon l'une quelconque des revendications précédentes, **caractérisée en ce que** le dérivé de l'acide bisphosphonique est choisi parmi :
- l'acide 1-hydroxy-éthylidène bisphosphonique et ses sels de sodium;
- l'acide 2-pyridin-2-yl-éthylidène bisphosphonique et ses sels de sodium;
- l'acide dichlorométhylène bisphosphonique et ses sels de sodium;
- l'acide 3-amino-1-hydroxy-propylidène bisphosphonique et ses sels de sodium;
- l'acide 4-amino-1-hydroxy-butylidène bisphosphonique et ses sels de sodium;
- l'acide 6-amino-1-hydroxy-hexylidène bisphosphonique et ses sels;
- l'acide phénoxyméthylène bisphosphonique et ses sels;
- l'acide thiomorpholinométhylène bisphosphonique et ses sels;
- l'acide 4-chloro-phénylthiométhylène bisphosphonique et ses sels;
- l'acide 1-hydroxy-2-(pyridin-3-yl)éthylidène bisphosphonique et ses sels de sodium;
- l'acide 1-hydroxy-2-(imidazol-2-yl)éthyl-1,1- bisphosphonique et ses sels;
- l'acide (cycloheptylamino)méthylène bisphosphonique et ses sels;
- l'acide 2-hydroxyéthylidène-2-(pyridin-3-yl)-1,1- bisphosphonique et ses sels de sodium.

10. Utilisation selon la revendication 9, **caractérisée en ce que** le dérivé de l'acide bisphosphonique est l'acide 4-chloro-phénylthiométhylène bisphosphonique.

## Claims

1. Use of a bisphosphonic acid derivative of the formula: 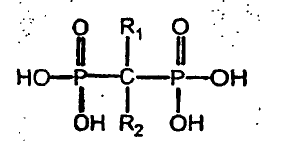 in which:
- R₁ represents a hydrogen atom, a halogen atom, a hydroxyl, an amino, a mono(C₁-C₄)alkylamino, a di (C₁-C₄) alkylamino;
- R₂ represents a halogen atom, a linear alkyl comprising from 1 to 5 carbon atoms, unsubstituted or substituted by a group selected from among a chlorine atom, a hydroxyl, an amino, a mono (C₁-C₄) alkylamino, a di (C₁-C₄) alkylamino; a cycle (C₃-C₇) alkylamino,
or R₂ represents a phenoxy, a phenyl, a thiol, a phenylthio, a chlorophenylthio, pyridyl, a pyridylmethyl, a 1-pyridyl-1-hydroxymethyl, a imidazolylmethyl, a thiomorpholin-4-yl of one of the pharmaceutically acceptable salts thereof or of one the hydrates thereof in the preparation of a medicine for treating lameness associated with osteoarthritis, osteochondrosis or enthesopathy of the bone insertions of tendons, ligaments or aponeuroses an animal not suffering from arthritis or fractures or exostoses.

2. Use according to claim 1, **characterised in that** the animal belongs to the family of the Equidae.

3. Use according to claim 1, **characterised in that** the animal is the horse.

4. Use according to any one of claims 1 to 3 for the preparation of a medicine containing from 0.001 mg to 10 g of the bisphosphonic acid derivative per dosage unit.

5. Use according to any one of the preceding claims, **characterised in that** the medicine is an orally or parenterally administrable liquid preparation containing from 0.001% to 90% by weight/volume of the bisphosphonic acid derivative.

6. Use according to any one of claims 1 to 4 for the preparation of an orally administrable medicine.

7. Use according to any one of claims 1 to 4 for the preparation of a parenterally administrable medicine.

8. Use according to any one of claims 1 to 4 for the preparation of a medicine administrable in the form of an implant.

9. Use according to any one of the preceding claims, **characterised in that** the bisphosphonic acid derivative is selected from among:
- 1-hydroxy-ethylidene bisphosphonic acid and the sodium salts thereof;
- 2-pyridin-2-yl-ethylidene bisphosphonic acid and the sodium salts thereof;
- dichloromethylene bisphosphonic acid and the sodium salts thereof;
- 3-amino-1-hydroxy-propylidene bisphosphonic acid and the sodium salts thereof;
- 4-amino-1-hydroxy-butylidene bisphosphonic acid and the sodium salts thereof;
- 6-amino-1-hydroxy-hexylidene bisphosphonic acid and the salts thereof;
- phenoxymethylene bisphosphonic acid and the salts thereof;
- thiomorpholinomethylene bisphosphonic acid and the salts thereof;
- 4-chlorophenylthiomethylene bisphosphonic acid and the salts thereof;
- 1-hydroxy-2-(pyridin-3-yl)ethylidene bisphosphonic acid and the sodium salts thereof;
- 1-hydroxy-2-(imidazol-2-yl)ethyl-1,1-bisphosphonic acid and the salts thereof;
- (cycloheptylamino)methylene bisphosphonic acid and the salts thereof;
- 2-hydroxyethylidene-2-(pyridin-3-yl)-1,1-bisphosphonic acid and the sodium salts thereof.

10. Use according to claim 9, **characterised in that** the bisphosphonic acid derivative is 4-chlorophenylthiomethylene bisphosphonic acid.

## Patentansprüche

1. Verwendung eines Derivats der Biphosphonsäure mit der Formel: 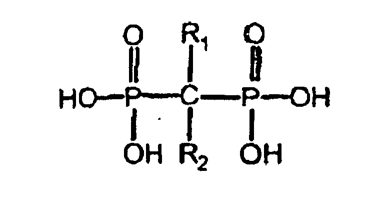 worin:
- R₁ ein Wasserstoffatom, ein Halogenatom, eine Hydroxylgruppe, eine Aminogruppe, eine C₁-C₄-Monoalkylaminogruppe, eine C₁-C₄-Dialkylaminogruppe darstellt;
- R₂ ein Halogenatom, eine lineare Alkylgruppe mit 1 bis 5 Kohlenstoffatomen, die unsubstituiert oder mit einer Gruppe substituiert ist, ausgewählt aus einem Chloratom, einer Hydroxylgruppe, einer Aminogruppe, einer C₁-C₄-Monoalkylaminogruppe, einer C₁-C₄-Dialkylaminogruppe, einer C₃-C₇-Cycloalkylaminogruppe, darstellt,
oder R₂ eine Phenoxy-, Phenyl-, Thiol-, Phenylthio, Chlorphenylthio-, Pyridyl-, Pyridylmethyl-, 1-Pyridyl-1-hydroxymethyl-, Imidazolylmethyl-, eine Thiomorpholin-4-yl-Gruppe darstellt;
eines seiner pharmazeutisch zulässigen Salze oder eines seiner Hydrate bei der Herstellung eines Medikaments, das zur Behandlung von Hinkbewegungen bestimmt ist, welche bei einer Osteoarthrose, einer Osteochondrose oder bei Enthesiopathien der Knocheninsertion von Sehnen, Bändern oder den Aponeurosen bei einem Tier auftreten, welches nicht an Arthritis, Brüchen oder Exostosen leidet.

2. Verwendung nach Anspruch 1, **dadurch gekennzeichnet, dass** das Tier der Familie der Einhufer angehört.

3. Verwendung nach Anspruch 1, **dadurch gekennzeichnet, dass** das Tier ein Pferd ist.

4. Verwendung nach einem der Ansprüche 1 bis 3 für die Herstellung eines Medikaments, welches 0,001 mg bis 10 g des Derivats der Biphosphonsäure pro Dosierungseinheit enthält.

5. Verwendung nach einem der vorhergehenden Ansprüche, **dadurch gekennzeichnet, dass** das Medikament ein flüssiges Präparat ist, das oral oder parenteral verabreicht werden kann und 0,001 Vol.-% bis 90 Vol.-% des Derivats der Biphosphonsäure enthält.

6. Verwendung nach einem der Ansprüche 1 bis 4 für die Herstellung eines Medikaments, welches oral verabreicht werden kann.

7. Verwendung nach einem der Ansprüche 1 bis 4 für die Herstellung eines Medikaments, welches parenteral verabreicht werden kann.

8. Verwendung nach einem der Ansprüche 1 bis 4 für die Herstellung eines Medikaments, welches in Form eines Implantats verabreicht werden kann.

9. Verwendung nach einem der vorhergehenden Ansprüche, **dadurch gekennzeichnet, dass** das Derivat der Biphosphonsäure ausgewählt wird aus:
- 1-Hydroxyethylidenbiphosphonsäure und deren Natriumsalze;
- 2-Pyridin-2-ylethylidenbiphosphonsäure und deren Natriumsalze;
- Dichlormethylenbiphosphonsäure und deren Natriumsalze;
- 3-Amino-1-hydroxypropylidenbiphosphonsäure und deren Natriumsalze;
- 4-Amino-1-hydroxybutylidenbiphosphonsäure und deren Natriumsalze;
- 6-Amino-1-hydroxyhexylidenbiphosphonsäure und deren Salze;
- Phenoxymethylenbiphosphonsäure und deren Salze;
- Thiomorpholinomethylenbiphosphonsäure und deren Salze;
- 4-Chlorphenylthiomethylenbiphosphonsäure und deren Salze;
- 1-Hydroxy-2-(pyridin-3-yl)ethylidenbiphosphonsäure und deren Natriumsalze;
- 1-Hydroxy-2-(imidazol-2-yl)ethyl-1,1-biphosphonsäure und deren Salze;
- (Cycloheptylamino)methylenbiphosphonsäure und deren Salze;
- 2-Hydroxyethyliden-2-(pyridin-3-yl)-1,1-biphosphonsäure und deren Natriumsalze.

10. Verwendung nach Anspruch 9, **dadurch gekennzeichnet, dass** das Derivat der Biphosphonsäure 4-Chlorphenylthiomethylenbiphosphonsäure ist.
